# EUROPEAN PATENT APPLICATION

(11) **EP 3 643 261 A1**
(43) Date of publication of application: **29.04.2020**
(21) Application number: 19204535.9
(22) Date of filing: 22.10.2019
(51) Int. Cl.: A61B 17/32

(54) **OSCILLATING SURGICAL CUTTING INSTRUMENT AND METHOD**

(30) Priority: 23.10.2018 US 201862749484 P; 26.09.2019 US 201916584053
(71) Applicant: Covidien LP, Mansfield, MA 02048 (US)
(72) Inventor: BEGG, Nikolai D., Wellesley, MA 02481 (US)
(74) Representative: Maschio, Antonio

(57) **Abstract**

An oscillating surgical cutting instrument includes a housing, a blade with at least a portion positioned distally of the housing, a transducer at least partially disposed within the housing, and a waveguide interconnecting the transducer and the blade. The transducer, waveguide, and blade are configured such that mechanical vibration energy produced by the transducer oscillates the blade at a frequency or within a frequency range from about 20 Hz to about 20 KHz to facilitate tissue dissection while minimizing thermal effects on tissue. A method of mechanical tissue dissection includes oscillating a blade at a frequency or within a frequency range from about 20 Hz to about 20 KHz and urging a tissue-contacting surface of the blade into contact with tissue to mechanically dissect tissue while minimizing thermal effects on tissue.

## Description

### BACKGROUND

### 1. Technical Field

The present disclosure relates generally to surgical instruments and methods. In particular, the present disclosure relates to an oscillating surgical cutting instrument and method for mechanically cutting tissue while minimizing thermal effects on tissue, e.g., cavitation, coagulation, thermal damage, etc.

### 2. Background of Related Art

Tissue dissection is a surgical task performed in many surgical procedures such as, for example, to remove tissue or to provide access to underlying tissue. Mechanical tissue dissection may be generally classified as either blunt tissue dissection or sharp tissue dissection. As the names suggest, blunt tissue dissection is effected by applying mechanical force to tissue to separate tissue without slicing, whereas sharp tissue dissection involves slicing to separate tissue.

Since mechanical tissue dissection can be a tedious process, energy is often employed to assist in tissue dissection by heating tissue to cavitate, coagulate, or otherwise treat tissue with energy, e.g., RF energy, microwave energy, thermal energy, laser energy, ultrasonic energy, etc. However, treating tissue in this manner produces thermal effects on tissue (cavitation, coagulation, thermal damage, etc.).

It would therefore be useful to provide a surgical cutting instrument and method that facilitates tissue dissection while minimizing thermal effects on tissue.

### SUMMARY

As used herein, the term "distal" refers to the portion that is being described which is further from a user, while the term "proximal" refers to the portion that is being described which is closer to a user. Further, to the extent consistent, any of the aspects described herein may be used in conjunction with any or all of the other aspects described herein.

Provided in accordance with aspects of the present disclosure is an oscillating surgical cutting instrument including a housing, a blade, a transducer, and a waveguide. At least a portion of the blade is positioned distally of the housing. The transducer is at least partially disposed within the housing and configured to convert electrical energy into mechanical vibration energy. The waveguide interconnects the transducer and the blade such that the mechanical vibration energy produced by the transducer is transmitted along the waveguide to the blade to oscillate the blade. The transducer, waveguide, and blade are configured such that the mechanical vibration energy produced by the transducer oscillates the blade at a frequency or within a frequency range from about 20 Hz to about 20 KHz to facilitate tissue dissection while minimizing thermal effects on tissue.

In an aspect, the mechanical vibration energy produced by the transducer oscillates the blade at a frequency or within a frequency range from about 500 Hz to about 20 KHz. In another aspect, the mechanical vibration energy produced by the transducer oscillates the blade at a frequency or within a frequency range from about 2 KHz to about 20 KHz. In still another aspect, the mechanical vibration energy produced by the transducer oscillates the blade at a frequency or within a frequency range from about 6 KHz to about 20 KHz. In yet another aspect, the mechanical vibration energy produced by the transducer oscillates the blade at a frequency or within a frequency range from about 20 Hz to about 16 KHz. In still yet another aspect, the mechanical vibration energy produced by the transducer oscillates the blade at a frequency or within a frequency range from about 20 Hz to about 12 KHz. In another aspect, the mechanical vibration energy produced by the transducer oscillates the blade at a frequency or within a frequency range from about 20 Hz to about 8 KHz. In yet another aspect, the mechanical vibration energy produced by the transducer oscillates the blade at a frequency or within a frequency range from about 500 Hz to about 16 KHz. In still another aspect, the mechanical vibration energy produced by the transducer oscillates the blade at a frequency or within a frequency range from about 2 KHz to about 12 KHz.

In aspects of the present disclosure, the blade defines at least one sharp portion configured for sharp tissue dissection and/or at least one blunt portion configured for blunt tissue dissection.

In aspects of the present disclosure, the transducer is a voice coil actuator.

A method of mechanical tissue dissection provided in accordance with aspects of the present disclosure includes oscillating a blade at a frequency or within a frequency range from about 20 Hz to about 20 KHz and urging a tissue-contacting surface of the blade into contact with tissue to mechanically dissect tissue while minimizing thermal effects on tissue.

In aspects of the present disclosure, urging the tissue-contacting surface into contact with tissue include urging a blunt or sharp portion of the blade into contact with tissue.

In an aspect of the present disclosure, oscillating the blade includes oscillating the blade at a frequency or within a frequency range from about 500 Hz to about 20 KHz. In another aspect, oscillating the blade includes oscillating the blade at a frequency or within a frequency range from about 2 KHz to about 20 KHz. In yet another aspect, oscillating the blade includes oscillating the blade at a frequency or within a frequency range from about 20 Hz to about 16 KHz. In still another aspect, oscillating the blade includes oscillating the blade at a frequency or within a frequency range from about 20 Hz to about 12 KHz.

### BRIEF DESCRIPTION OF THE DRAWINGS

Various aspects and features of the present disclosure are described hereinbelow with reference to the lone drawing, which is a perspective view of an oscillating surgical cutting instrument provided in accordance with the present disclosure.

### DETAILED DESCRIPTION

The present disclosure provides an oscillating surgical cutting instrument 100 and method that facilitates tissue dissection while minimizing thermal effects on tissue. Although oscillating surgical cutting instrument 100 is illustrated and described herein as a pencil-style device, the present disclosure it not limited to this configuration; rather, it is contemplated that the aspects and features of the present disclosure may also be embodied in a pistol-grip-style device, a shaft-based device, an attachment for use with a surgical robot, any other suitable device, and/or may be a stand-alone device or may be coupled to another device (integrally therewith or as a removable accessory). Regardless of the particular configuration, the aspects and features of the presently disclosed oscillating surgical cutting instrument 100 that facilitate tissue dissection while minimizing thermal effects on tissue remain generally consistent.

Instrument 100 generally includes a housing 110, a waveguide 120 extending through housing 110, a blade 130 coupled to waveguide 120 and extending distally from housing 110, one or more activation buttons 140 operably disposed on housing 110, a transducer 150 disposed within housing 110 and operably coupled to waveguide 120 for producing mechanical vibrations transmitted along waveguide 120 to blade 130, and a plug assembly 160 adapted to connect instrument 100 to an energy source (not shown), e.g., a power receptacle or a surgical generator, for driving transducer 150. As an alternative to plug assembly 160, transducer 150 may be battery-powered via an onboard battery (not shown), e.g., disposed within or coupled to housing 110. At least a portion of housing 110 may be configured as a handle to facilitate grasping and manipulation by a user.

Waveguide 120 and blade 130 may be monolithically-formed from a single piece of material or may be separate components permanently or removably coupled to one another. Blade 130 may define a spatula-shaped configuration having opposing relatively wide sides 132 and opposing relatively narrow sides 134, as illustrated, or may define any other suitable configuration such as, for example, a ball shape, hook (or other curved) shape, angled shape (including one or more angled sections), cylindrical shape, elongated polygonal shape, combinations thereof, etc., and may have one or more blunt tissue-contacting surfaces and/or one or more sharp tissue-contacting edges or points. Blade 130 is configured to be maneuvered into position such that a tissue-contacting surface thereof is in contact with tissue and, as detailed below, to oscillate to facilitate dissection, e.g., blunt or sharp dissection, of tissue in contact therewith while minimizing thermal effects on tissue.

Blade 130 may be configured and/or driven (based on the configuration of waveguide 120 and/or the output of transducer 150) to oscillate in one axial direction, e.g., longitudinally, in two axial directions, e.g., longitudinally and in one transverse axial direction, or in all three axial directions, e.g., longitudinally and in both transverse axial directions. With respect to oscillation in two axial direction or all three axial directions, for example, blade 130 may be configured and/or driven to oscillate in an elliptical or ellipsoidal pattern.

Transducer 150 may be any suitable device capable of converting electrical energy supplied from the energy source (not shown) into mechanical vibration energy for transmission along waveguide 120 to blade 130 to oscillate blade 130. More specifically, in embodiments, transducer 150 is a voice coil actuator (VCA). In other embodiments, transducer 150 may be a solenoid, a piezoelectric transducer, or other suitable transducer. Regardless of the particular configuration of transducer 150, transducer 150, waveguide 120, and blade 130 are configured such that mechanical vibration energy produced by transducer 150 is transmitted along waveguide 120 to blade 130 to oscillate blade 130 at a frequency or within a frequency range that facilitates mechanical tissue dissection (blunt or sharp) while minimizing thermal effects on tissue.

It has been found that oscillating blade 130 at a frequency or within a frequency range in the audible range, from about 20 Hz to about 20 KHz (wherein "about" takes into account manufacturing, system, and measurement tolerances) facilitates mechanical tissue dissection while minimizing thermal effects on tissue. More specifically, by maintaining the frequency or frequency range in the sub-ultrasonic frequency range, below 20 KHz, the mechanical vibration energy imparted to blade 130 facilitates tissue dissection while minimizing thermal effects on tissue, e.g., minimizing heating tissue to the point of irreversible thermal effect.

In embodiments, the frequency or frequency range is at or below about 20 KHz; in other embodiments, at or below about 16 KHz; in still other embodiments, at or below about 12 KHz; and in yet other embodiments, at or below 8 KHz. In embodiments, in conjunction with any of the above-noted upper limits, the frequency or frequency range is at or above about 20 Hz; in other embodiments, at or above about 500 Hz; in still other embodiments, at or above about 2 KHz; in yet other embodiments, at or above about 6 KHz.

Activation buttons 140 are operably disposed on housing 110, as noted above, and one or more of activation buttons 140 is disposed in communication with transducer 150 to enable user-selected activation, deactivation, and/or mode selection. For example, in embodiments, one activation button 140 is selectively depressible to activate transducer 150 to drive blade 130 to oscillate in a low power (lower amplitude and/or frequency) mode while another activation button 140 is selectively depressible to activate transducer 150 to drive blade 130 to oscillate in a high power (higher amplitude and/or frequency) mode. Multi-stage buttons are additionally or alternatively contemplated.

Plug assembly 160, as noted above, is adapted to connect instrument 100 to an energy source (not shown), e.g., a power receptacle or a surgical generator, for driving transducer 150. Plug assembly 160, more specifically, includes a plug 162 adapted to mechanically and electrically connect to the energy source and a cable 164 housing one or more electrical leads 166 for communicating electrical energy to transducer 150 to power transducer 150, e.g., in response to activation or one or more of activation buttons 140. In embodiments where electrosurgical energy is also provided, as detailed below, one or more electrical leads 166 of plug assembly 160 may be configured to communicate electrosurgical energy to the electrode portion(s) 170 of blade 130 for energy-based tissue treatment.

In embodiments, in addition to oscillating blade 130 at a sub-ultrasonic frequency or within a sub-ultrasonic frequency range to facilitate mechanical tissue dissection, blade 130 may include one or more electrode portions 170 adapted to connect to a source of electrosurgical energy, e.g., RF energy, for energy-based tissue treatment, e.g., coagulation or electrical/electromechanical dissection. The electrode portion(s) 170 may be electrically-conductive section(s) of blade 130 and/or electrically-conducive component(s), e.g., plate(s), wire(s), coating(s), etc., disposed on, in, and/or otherwise associated with blade 130. In other embodiments, the entirety of blade 130 is electrically-conductive.

One of activation buttons 140 may be selectively depressible to initiate the supply of energy to the electrode portion(s) 170 of blade 130. Energy-based tissue treatment may be effected in a monopolar manner, e.g., wherein energy is transmitted from the electrode portion 170 to a remote return pad or separate return device (not shown), or may be effected in bipolar manner, e.g., wherein energy is conducted between isolated electrode portions 170 defining an electrical potential gradient therebetween. Electrosurgical energy may be supplied to the electrode portion(s) 170 of blade 130 in conjunction with oscillation of blade 130 or separately therefrom.

As an alternative or in addition to electrosurgical energy application, surgical instrument 100 may be configured to oscillate blade 130 at ultrasonic frequencies or within an ultrasonic frequency range. More specifically, in embodiments, one or more of activation buttons 140 may be configured to drive oscillation of blade 130 at an ultrasonic frequency or within an ultrasonic frequency range, above about 20 KHz, for energy-based tissue treatment. In such embodiments, transducer 150 may be configured to provide both the sub-ultrasonic frequency oscillation of blade 130 (to dissect tissue with minimal thermal effect) and the ultrasonic frequency oscillation of blade 130 (to provide energy-based tissue treatment, e.g., coagulation or electrical/electromechanical dissection). Alternatively, a separate transducer (not shown) may be provided for enabling oscillation of blade 130 at an ultrasonic frequency or within an ultrasonic frequency range. In embodiments, when operating in the ultrasonic frequency range, the frequency or range is about 40 KHz to about 65 KHz; in other embodiments about 45 KHz to about 60 KHz; and in other embodiments about 50 KHz to about 55 KHz.

With respect to methods provided in accordance with the present disclosure, oscillating a surgical blade (blunt and/or sharp) at a frequency or within a frequency range in the sub-ultrasonic frequency range, e.g., according to any of the limits and/or ranges detailed above, and urging the surgical blade into contact with the tissue to be dissected is utilized to facilitate mechanical tissue dissection while minimizing thermal effects on tissue. Electrosurgical and/or ultrasonic energy application may be utilized where energy-based tissue treatment is desired.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs: -
1. An oscillating surgical cutting instrument, comprising:
   a housing;
   a blade, at least a portion of the blade positioned distally of the housing;
   a transducer at least partially disposed within the housing and configured to convert electrical energy into mechanical vibration energy; and
   a waveguide interconnecting the transducer and the blade such that the mechanical vibration energy produced by the transducer is transmitted along the waveguide to the blade to oscillate the blade,
   wherein the transducer, waveguide, and blade are configured such that the mechanical vibration energy produced by the transducer oscillates the blade at a frequency or within a frequency range from about 20 Hz to about 20 KHz to facilitate tissue dissection while minimizing thermal effects on tissue.
2. The oscillating surgical cutting instrument according to paragraph 1, wherein the mechanical vibration energy produced by the transducer oscillates the blade at a frequency or within a frequency range from about 500 Hz to about 20 KHz.
3. The oscillating surgical cutting instrument according to paragraph 1, wherein the mechanical vibration energy produced by the transducer oscillates the blade at a frequency or within a frequency range from about 2 KHz to about 20 KHz.
4. The oscillating surgical cutting instrument according to paragraph 1, wherein the mechanical vibration energy produced by the transducer oscillates the blade at a frequency or within a frequency range from about 6 KHz to about 20 KHz.
5. The oscillating surgical cutting instrument according to paragraph 1, wherein the mechanical vibration energy produced by the transducer oscillates the blade at a frequency or within a frequency range from about 20 Hz to about 16 KHz.
6. The oscillating surgical cutting instrument according to paragraph 1, wherein the mechanical vibration energy produced by the transducer oscillates the blade at a frequency or within a frequency range from about 20 Hz to about 12 KHz.
7. The oscillating surgical cutting instrument according to paragraph 1, wherein the mechanical vibration energy produced by the transducer oscillates the blade at a frequency or within a frequency range from about 20 Hz to about 8 KHz.
8. The oscillating surgical cutting instrument according to paragraph 1, wherein the mechanical vibration energy produced by the transducer oscillates the blade at a frequency or within a frequency range from about 500 Hz to about 16 KHz.
9. The oscillating surgical cutting instrument according to paragraph 1, wherein the mechanical vibration energy produced by the transducer oscillates the blade at a frequency or within a frequency range from about 2 KHz to about 12 KHz.
10. The oscillating surgical cutting instrument according to paragraph 1, wherein the blade defines at least one sharp portion configured for sharp tissue dissection.
11. The oscillating surgical cutting instrument according to paragraph 1, wherein the blade defines at least one blunt portion configured for blunt tissue dissection.
12. The oscillating surgical cutting instrument according to paragraph 1, wherein the blade defines at least one sharp portion configured for sharp tissue dissection and at least one blunt portion configured for blunt tissue dissection.
13. The oscillating surgical cutting instrument according to paragraph 1, wherein the transducer is a voice coil actuator.
14. A method of mechanical tissue dissection, comprising:
   oscillating a blade at a frequency or within a frequency range from about 20 Hz to about 20 KHz; and
   urging a tissue-contacting surface of the blade into contact with tissue to mechanically dissect tissue while minimizing thermal effects on tissue.
15. The method according to paragraph 14, wherein urging the tissue-contacting surface into contact with tissue include urging a blunt portion of the blade into contact with tissue.
16. The method according to paragraph 14, wherein urging the tissue-contacting surface into contact with tissue include urging a sharp portion of the blade into contact with tissue.
17. The method according to paragraph 14, wherein oscillating the blade includes oscillating the blade at a frequency or within a frequency range from about 500 Hz to about 20 KHz.
18. The method according to paragraph 14, wherein oscillating the blade includes oscillating the blade at a frequency or within a frequency range from about 2 KHz to about 20 KHz.
19. The method according to paragraph 14, wherein oscillating the blade includes oscillating the blade at a frequency or within a frequency range from about 20 Hz to about 16 KHz.
20. The method according to paragraph 14, wherein oscillating the blade includes oscillating the blade at a frequency or within a frequency range from about 20 Hz to about 12 KHz.

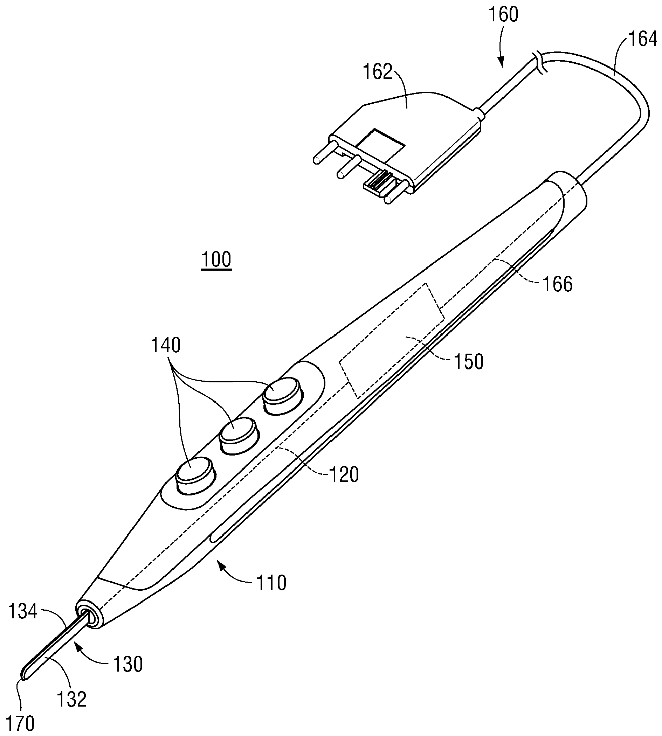

## Claims

1. An oscillating surgical cutting instrument, comprising:
a housing;
a blade, at least a portion of the blade positioned distally of the housing;
a transducer at least partially disposed within the housing and configured to convert electrical energy into mechanical vibration energy; and
a waveguide interconnecting the transducer and the blade such that the mechanical vibration energy produced by the transducer is transmitted along the waveguide to the blade to oscillate the blade,
wherein the transducer, waveguide, and blade are configured such that the mechanical vibration energy produced by the transducer oscillates the blade at a frequency or within a frequency range from about 20 Hz to about 20 KHz to facilitate tissue dissection while minimizing thermal effects on tissue.

2. The oscillating surgical cutting instrument according to claim 1, wherein the mechanical vibration energy produced by the transducer oscillates the blade at a frequency or within a frequency range from about 500 Hz to about 20 KHz.

3. The oscillating surgical cutting instrument according to claim 1, wherein the mechanical vibration energy produced by the transducer oscillates the blade at a frequency or within a frequency range from about 2 KHz to about 20 KHz.

4. The oscillating surgical cutting instrument according to claim 1, wherein the mechanical vibration energy produced by the transducer oscillates the blade at a frequency or within a frequency range from about 6 KHz to about 20 KHz.

5. The oscillating surgical cutting instrument according to claim 1, wherein the mechanical vibration energy produced by the transducer oscillates the blade at a frequency or within a frequency range from about 20 Hz to about 16 KHz.

6. The oscillating surgical cutting instrument according to claim 1, wherein the mechanical vibration energy produced by the transducer oscillates the blade at a frequency or within a frequency range from about 20 Hz to about 12 KHz.

7. The oscillating surgical cutting instrument according to claim 1, wherein the mechanical vibration energy produced by the transducer oscillates the blade at a frequency or within a frequency range from about 20 Hz to about 8 KHz.

8. The oscillating surgical cutting instrument according to claim 1, wherein the mechanical vibration energy produced by the transducer oscillates the blade at a frequency or within a frequency range from about 500 Hz to about 16 KHz.

9. The oscillating surgical cutting instrument according to claim 1, wherein the mechanical vibration energy produced by the transducer oscillates the blade at a frequency or within a frequency range from about 2 KHz to about 12 KHz.

10. The oscillating surgical cutting instrument according to any preceding claim, wherein the blade defines at least one sharp portion configured for sharp tissue dissection.

11. The oscillating surgical cutting instrument according to any preceding claim, wherein the blade defines at least one blunt portion configured for blunt tissue dissection.

12. The oscillating surgical cutting instrument according to any preceding claim, wherein the blade defines at least one sharp portion configured for sharp tissue dissection and at least one blunt portion configured for blunt tissue dissection.

13. The oscillating surgical cutting instrument according to any preceding claim, wherein the transducer is a voice coil actuator.
